# EUROPEAN PATENT SPECIFICATION

(11) **EP 4 151 084 B1**
(45) Date of publication and mention of the grant of the patent: **29.10.2025**
(21) Application number: 22194757.5
(22) Date of filing: 09.09.2022
(51) Int. Cl.: A01H 6/88, C12N 9/22, C12N 15/82, A01H 5/08

(54) **TRANSFECTION OF PROTOPLASTS FROM GRAPEVINE PLANTS WITH THE CAS9/GRNA COMPLEX AND REGENERATION OF RELATIVE EDITED GRAPEVINE PLANTS**
TRANSFEKTION VON PROTOPLASTEN AUS WEINREBEN MIT DEM CAS9/GRNA-KOMPLEX UND REGENERATION VON ENTSPRECHEND EDITIERTEN WEINREBEN
TRANSFECTION DE PROTOPLASTES DE PLANTS DE VIGNE AVEC LE COMPLEXE CAS9/GRNA ET RÉGÉNÉRATION DE PLANTS DE VIGNE ÉDITÉS EN RELATIF

(30) Priority: 10.09.2021 IT 202100023468
(43) Date of publication of application: 22.03.2023
(73) Proprietor: EdiVite S.r.l., 35020 San Pietro Viminario (PD) (IT)
(72) Inventor: Bertini, Edoardo, 37049 Villa Bartolomea (VR) (IT); Zenoni, Sara, 37036 San Martino Buon Albergo (VR) (IT); Tornielli, Giovanni Battista, 35127 Padova (IT)
(74) Representative: Münchow, Vera Ute Barbara

(56) References cited:
- MALNOY MICKAEL ET AL: "DNA-Free Genetically Edited Grapevine and Apple Protoplast Using CRISPR/Cas9 Ribonucleoproteins", FRONTIERS IN PLANT SCIENCE, vol. 7, 20 December 2016 (2016-12-20), CH, XP055779864, ISSN: 1664-462X, DOI: 10.3389/fpls.2016.01904
- OSAKABE YURIKO ET AL: "CRISPR-Cas9-mediated genome editing in apple and grapevine", NATURE PROTOCOLS, NATURE PUBLISHING GROUP, GB, vol. 13, no. 12, 2 November 2018 (2018-11-02), pages 2844 - 2863, XP036643354, ISSN: 1754-2189, [retrieved on 20181102], DOI: 10.1038/S41596-018-0067-9
- SCINTILLA SIMONE ET AL: "Regeneration of Plants from DNA-free Edited Grapevine Protoplasts", BIORXIV, 16 July 2021 (2021-07-16), XP055910764, Retrieved from the Internet <URL:https://www.biorxiv.org/content/10.1101/2021.07.16.452503v1.full.pdf> [retrieved on 20220408], DOI: 10.1101/2021.07.16.452503
- PARK SUNG-CHUL ET AL: "DNA-free mutagenesis ofinvar.using CRISPR/Cas9 ribonucleoprotein complexes", PLANT BIOTECHNOLOGY REPORTS, SPRINGER JAPAN, JP, vol. 13, no. 5, 1 October 2019 (2019-10-01), pages 483 - 489, XP037044394, ISSN: 1863-5466, [retrieved on 20191025], DOI: 10.1007/S11816-019-00585-6

## Description

### TECHNICAL FIELD

The invention relates to the transfection of protoplasts from grapevine plants and the subsequent regeneration of the relative edited grapevine plants edited from the transfected protoplasts. The described protocols particularly relate to the field of genomic editing to combat diseases in plants while maintaining the identity and organoleptic profile of grapevine fruits.

### BACKGROUND ART

Maintaining the qualitative characteristics and the great variety of the ampelographic heritage typical of each area dedicated to viticulture, also giving the most valuable grape varieties resistance characteristics, is a demand that emerges from every part of the vine-growing sector and can be addressed by the most modern and rapid genetic improvement methods. The genome editing mediated by the CRISPR/Cas9 system has opened new perspectives and its application to the grapevine would make what is impossible for traditional breeding possible: obtaining a resistant clone of a traditional grapevine without changing its identity and the organoleptic profile of its fruits. From a biotechnological point of view, the application of genome editing for the production of a modified grapevine clone is not immediate and requires overcoming some technical obstacles. The mutation which is obtained by genome editing is in fact carried out by the CRISPR/Cas9 complex inside the cell, in which it can be inserted, in particular by eliminating the wall, if it is not to be inserted by means of stable genetic modification or bombardment methods.

The wall removal starting from plant embryogenetic cells produces a protoplast, which must maintain the ability to regenerate the plant. Such an ability has been demonstrated in a limited number of scientific works and in particular for protoplasts derived from embryogenic material obtained from different tissues (Reustle et al., 1995; Zhu et al., 1997; Bertini et al., 2019).

The possibility of introducing the CRISPR/Cas9 complex inside protoplast cells and the subsequent generation of the mutation at the site of interest have also been demonstrated in grapevines (Malnoy et al., 2016; Osakabe et al., 2018). However, to date only one regeneration of edited grapevine plants obtained from protoplasts to which the CRISPR/Cas9 system has been applied (Scintilla et al. 2021) has been demonstrated.

### DISCLOSURE OF THE INVENTION

The invention aims to propose a method which allows to transfect protoplasts from grapevine plants and regenerate the corresponding edited grapevine plants from the protoplasts transfected with the Cas9/gRNA complex. A further object of the invention is to improve the step of transfecting protoplasts to reduce the stress to the protoplasts and increase the likelihood of regenerating the plants from the transfected protoplasts. Another object of the invention is to find alternatives to the state of the art to also improve the method for regenerating plants from protoplasts.

The object is achieved by a method for transfection of protoplasts from grapevine plants comprising the following steps:
(I) preparation of a Cas9:gRNA complex with a molar ratio of 1:3 - 3:1, in particular with a weight ratio between 1:1 and 3:1, and incubating the ribonucleoprotein complex in the dark;
(II) addition of the Cas9:gRNA complex prepared in the previous step to the protoplasts;
(III) addition of a PEG 3000 - PEG 5000 solution, preferably PEG 4000, to the mixture obtained in step (II) and incubation, preferably at room temperature, in the dark;
(IV) addition of a saline solution containing MES, in particular a W5 solution, stirring and incubation, preferably at room temperature, and preferably in the dark;
(V) at least once repetition of step (IV), in particular with the doubled volume of saline solution;
(VI) centrifugation of the transfected protoplasts washed in the previous steps preferably for 2 - 4 minutes at 80 - 120×g more preferably 3 min at 100×g and removal of the supernatant;
(VII) proceed with the cultivation of the protoplasts without further incubation with incubation periods exceeding the duration of 10 min, in particular without any incubation.

The above protocol allows a delicate transfection of the protoplasts in order to hardly stress them and make them suitable for the subsequent plant cultivation. gRNA is intended as any kind of guide RNA which functions as a guide for the Cas9 enzyme which targets RNA or DNA, with which it forms complexes. One or more component gRNAs, such as the cr/tracrRNA system, are conceivable. If incubations are to be performed in step (VII), these preferably occur without the addition of further washing solutions. Very advantageously, no further incubation occurs in step (VII), greatly reducing the stress for the protoplasts.

The various steps performed in the dark further reduce the stress for the protoplasts, an effect which makes them more "available" to plant cultivation.

In a preferred embodiment of the invention, the final volume of the mixture of step (I) is 15-25 µL and/or the incubation time in step (I) is 10 min. Higher volumes should preferably be avoided.

Preferably, the protoplasts for the addition of the Cas9-gRNA complex were resuspended in a concentration of 2 x 10⁵ protoplasts in 200 µL of a MMG solution and in step (III) 200 - 225 µL, preferably 200 µL of PEG 3000 - PEG 5000 solution, preferably PEG 4000, preferably at 40% by weight by volume, are added.

The above concentration is to be understood as concentration and can thus be expressed in concentrations referring to other volumes of solution (for example: 1 x 10⁵ protoplasts in 100 µL of a MMG solution), correspondingly varies the volume of added PEG solution. PEG 4000 gave the best results, even if the protocol works equally with PEGs of different molecular weight.

Dark conditions during PEG-treatment in DNA-free genome editing protocols in *Brassica oleracea* var. capitata have been described by Park et al in 2019.

Advantageously, under these particular conditions, in step (IV) a volume of saline solution containing MES is added, in particular a W5 solution, which is twice the volume of the MMG solution and for which the incubation time is about 8 - 12 min, in particular 10 min.

In an embodiment of the invention, in step (VII) a concentration of 1x10⁵ ppt/mL on *petri* is used.

Obtaining the protoplasts themselves allows the application of different methods also known in the state of the art. Different sources of cells are conceivable, such as for example also floral stamens. In a preferred embodiment of the method according to the invention, said protoplasts are obtained from the following steps:
(0-1) obtaining embryogenic calluses from apical leaves of plants grown *in vitro*;
(0-2) proliferation of embryogenic calluses and regeneration of somatic embryos;
(0-3) optionally transformation of the embryos with *Agrobacterium tumefaciens,* in order to introduce a reporter gene, in particular the reporter gene encoding green fluorescent protein (GFP);
(0-4) from embryos, possibly transgenic embryos, induction of embryogenic callus and isolation of protoplasts.

An alternative to the optional step (0-3) for quickly verifying the functionality and effectiveness of the method is the use of GFP-fused Cas9 proteins. Step (0-3) is omitted during the application of the system to introduce mutations which make plants resistant to certain diseases, increase the quality of their fruits etc. Without the application of step (0-3), the obtainable plant is not genetically transformed, but is an edited plant.

The possibility of obtaining plants from transfected protoplasts can be further improved by intervening on the steps of the method which relate to the preparation of protoplasts for the cultivation of seedlings. In this regard, in a preferred variant of the method according to the invention, the following preparatory steps are added for regenerating grapevine plants from the transfected protoplasts:
(VIII) a culture medium, preferably of the Nitsch type, is added to the transfected protoplasts, wherein the liquefied medium is preferably added at a temperature above 35°C and below 48°C;
(IX) the same volume of culture medium, preferably of the Nitsch type, in the liquid state is added to the culture medium after solidification;
(X) incubation of the protoplasts, preferably at 27-28°C, in the dark until regeneration of embryos at the cotyledonary stage;
(XI) germination of the embryos and subsequent development of the seedlings.

The concentrations and quantities applied play a significant role. Preferably, in step (VIII) 2 mL of culture medium is added to 800-µL of the protoplast solution and the medium is separated into two halves, after solidification the same amount of culture medium is added as a reserve, and in step (VIII) and (IX) the culture medium is preferably of the Nitsch type supplemented with 2 mg/L 1-naphthaleneacetic acid (NAA), 0.5 mg/L 6-benzylaminopurine (6-BAP), 0.3 M glucose, 0.09 M sucrose and 2 g/L gellan gum (pH 5,7) wherein in step (IX) the medium was supplemented with 0.3% activated carbon or similar, e.g., glutathione; where during step (X) the liquid medium was replaced every 10-20 days, preferably every 12-18 days with fresh medium as defined above but without glucose, after 3-4 months of culture, the somatic embryos at the cotyledonary stage derived from protoplasts, were transferred to a solid preferably of the Nitsch type supplemented with 30 g/L sucrose and 2 g/L gellan gum (pH 5,7) and kept in the dark for 3-5 weeks, preferably 4-5 weeks to allow complete germination. The indication of the amount of medium and of the protoplast solution is not to be only understood as an absolute value, but more a ratio that is maintained: 2 mL medium/800-µL protoplast solution is equivalent for example to 1 mL medium/400 µL protoplast solution.

Summing up, it can be seen that the invention achieves the intended objects, and in particular the invention proposes a method which allows to transfect protoplasts of grapevine plants and regenerate the corresponding edited grapevine plants from the protoplasts transfected with the Cas9/gRNA complex, this through the improvement of the protoplast transfection step to reduce the stress to the protoplasts and increase the probability of regenerating plants from transfected protoplasts and in a preferred embodiment by improving the method for regenerating plants from protoplasts.

Said objects and advantages will be further highlighted during the description of preferred embodiment examples of the invention given by way of example and not of limitation.

Variant embodiments of the invention are the object of the dependent claims. The description of the preferred embodiment examples according to the invention is given by way of nonlimiting example, in particular it is possible to replace the described features with equivalent features.

### DESCRIPTION OF PREFERRED EMBODIMENT EXAMPLES

- Figure 1: shows the apical leaf of the Thompson Seedless cultivar (A), the relative embryogenic callus (B), the somatic embryos transformed with the GFP gene (D), the relative embryogenic callus obtained from the transgenic somatic embryos (E).
- Figure 2: shows protoplasts isolated from Thompson Seedless embryogenic calluses over-expressing the GFP protein with white light (A) and UV light (B).
- Figure 3: shows the cotyledonary stage embryo in white light (A), the cotyledonary stage embryo in UV light (B), the regenerated plant in white light (C), and a detail of the plant in UV light (D).
- Figure 4: shows the Cas9/gRNA1 (RNP1) induced mutation on the GFP gene sequence.
- Figure 5: shows the Cas9/gRNA2 (RNP2) induced mutation on the GFP gene sequence.
- Figure 6: shows in an agarose gel electrophoresis analysis the outcome of *in-vitro* cutting for incubation in the light and incubation in the dark.
- Figure 7: shows on the left the Cas9/gRNA induced mutation in a Chardonnay variety and on the right the corresponding edited Chardonnay grapevine plant cultivated from edited protoplasts.

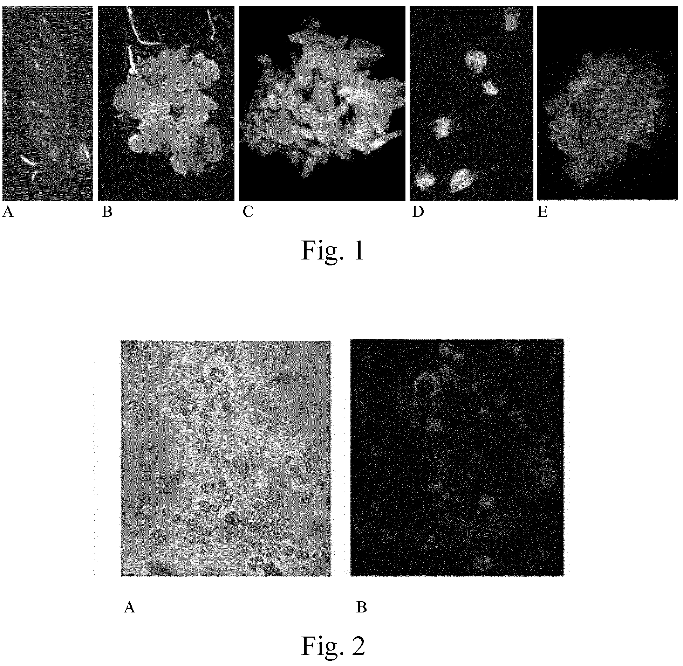

The following is a protocol applied to the Thompson Seedless variety. Embryogenic calluses were obtained from young apical leaves of plants grown *in vitro* (Figure 1A, B), following the protocol reported in the work of Dhekney et al. (2009). After proliferation of the embryogenic callus, somatic embryos were regenerated (Figure 1C). Those skilled in the art know other manners for producing somatic embryos.

The embryos were subsequently stably transformed with *Agrobacterium tumefaciens,* in order to introduce the reporter gene encoding green fluorescent protein (GFP) (Figure 1D). The protocol used to carry out the stable genetic transformation is that described by Li et al. (2008), already successfully applied to the cultivar in question. Those skilled in the art with their general knowledge easily identify other protocols for introducing the reporter gene also in other types of grapevines. A reporter gene is a gene whose activity is easily monitored by histochemistry or immunological methods; it encodes a gene product which can be used to study the activity of regulatory sequences of another gene of interest.

The expression of these genes is easily identifiable and quantifiable by simple assays, whereby they are used as selectable markers.

Reporter genes are therefore often used as indicators to assess the success of the transfection, i.e., highlight whether a certain gene has been acquired from the cells under consideration and whether the aforesaid gene is expressed correctly. It is therefore important that the reporter gene used is not naturally expressed in the cells of interest.

GFP was induced from over-expressing transgenic embryos with embryogenic callus standard methods (secondary embryogenesis) (Figure 1E) for the isolation of the protoplasts and the subsequent application of site-specific mutagenesis mediated by the CRISPR/Cas9 system. For the isolation and subsequent cultivation of the transgenic protoplasts over-expressing the GFP protein (Figure 2A, B), the protocol reported in Bertini et al. (2019) was used, which was improved in some points by the inventors. The modified protocol is applicable to any type of protoplast, whether modified with the CRISPR/Cas9 system or with other systems known in the state of the art, such as ZFN (Zinc Finger Nucleases), TALEN (Transcription activator-like effector nucleases) systems, etc.

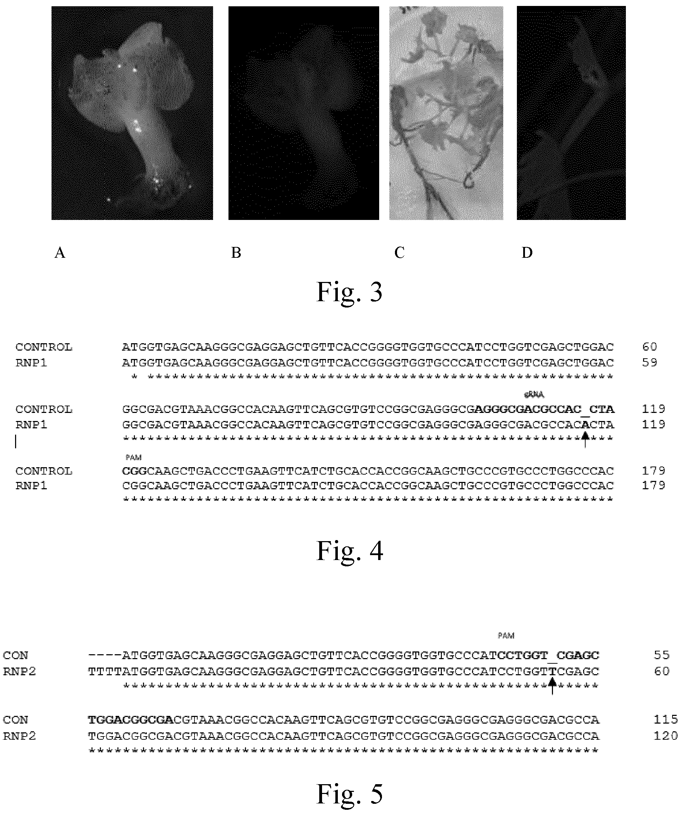

5×10⁵ - 1×10⁷ protoplasts (ppt) are extracted from 0.15 - 0.3 g of embryogenic callus. In order to calculate the regeneration efficiency, the Thompson Seedless protoplasts over-expressing GFP are subsequently cultivated at the concentration of 1×10⁵ ppt/mL. The regeneration efficiency is 50-100 cotyledonary stage embryos every 5×10⁵ - 5×10⁶ ppt after about 3 months of isolation (Figure 3A, B). The regeneration efficiency of the entire plant from cotyledonary stage embryos is 20-35% approximately 6 months after isolation (Figure 3C, D).

Thompson Seedless protoplasts over-expressing GFP are used for the application of the CRISPR-Cas9 system in order to mutate the sequence of the GFP encoding gene.

The software Cas-Designer (http://rgenome.net/) is used for the design of the guide RNA sequences (gRNA). The GeneArtTM Precision gRNA Synthesis Kit (Invitrogen, catalogue A29377) is used for the synthesis of guide RNAs.

The sequences designed on the GFP gene sequence and used for transfection are (the PAM sequence is highlighted in bold):
RNA guide 1: 5'-CGAGGGCGACGCCACCTA **CGG** -3' (SEQ. 1)
RNA guide 2: 5'-TCGCCGTCCAGCTCGACC **AGG** -3' (SEQ. 2)

The transfection of the protoplasts is performed with Cas9-gRNA ribonucleotides (RNPs). The scientific literature knows several protocols for performing the transfection of protoplasts, such as those described by Woo et al. (2015), optimized for Arabidopsis, rice, lettuce and tobacco, or Osakabe et al. (2018), optimized for apples and grapevines. Specifically, by grapevine the authors do not describe the regeneration of plants from protoplasts transformed with CRISPR/Cas9. With the protocol according to the invention, the regeneration efficiency of grapevine protoplasts has been greatly increased.

In a first step, the Cas9:gRNA complex was prepared with a ratio of 1:1 - 3:1 (w/v) in a final volume of 20-25 µL and the ribonucleoprotein complex was incubated at room temperature for 10 minutes in the dark.

2 x 10⁵ protoplasts were used for the transfection, resuspended in 200 µL MMG solution. The MMG solution preferably comprises 0,5 M mannitol, 4 mM MES (2-N-morpholino ethanesulfonic acid) and 15 mM MgCl₂ in distilled H₂O. RNP1 (gRNA1) and RNP2 (gRNA2), prepared in the previous step, are added to the protoplasts.

200 µL 40% (w/v) PEG 4000 solution was then added to the protoplast-RNP mixture. The mixing occurred by pipetting gently, for example with tips with the top cut to avoid breaking the cells, followed by incubation at room temperature for 20 minutes in the dark.

In a first wash 400 µL W5 solution was added, mixed and then incubated at room temperature for a further 10 minutes in the dark. A W5 solution comprises 5 mM glucose, 2 mM MES (pH 5.7), 154 mM NaCl, 125 mM CaCl₂, and 5 mM KCl in distilled H₂O. Advantageously, the solution is prepared fresh before use.

In a second wash, 800 µL W5 solution was added, mixed and then incubated again for 10 minutes at room temperature in the dark.

At the end of the washings, the transfected protoplasts were centrifuged for 3 minutes at 100×g and the supernatant was removed. To further reduce the stress, after centrifugation, the rest position was reached by inertia and not abruptly stopping the centrifugal machine.

Proceeding immediately with the cultivation of the protoplasts, for example at a concentration of 1µ10⁵ ppt/mL, without an incubation, in particular without an overnight incubation, a higher regeneration efficiency was observed.

For the subsequent cultivation of the protoplasts and the regeneration of embryos, the protocol described in Bertini et al. (2019) was used in modified form. In particular, the following modifications have been identified which can generally be applied to the regeneration of any type of plant from any type of protoplast, in particular *Vitis vinifera L.*

The entire method according to the invention, in particular the transfection of protoplasts, is transferable to other types of grapevines or plants.

Particular attention is paid to the amount of culture medium. 2 mL of culture medium are added to the transfected protoplasts, cultivated by the disc-culture method. To achieve the maximum homogeneity of the protoplasts in the solid culture medium, the liquefied medium is added at a temperature above 35°C and below 48°C.

The 2 mL are divided into two *petri* dishes, placing a 1 mL culture drop in each, adding the same volume of culture medium in the liquid state in each *petri* after solidification. By putting a single drop, the cells have greater contact with the liquid nutrient medium.

The plates are then incubated at 28°C in the dark until the regeneration of cotyledonary stage embryos.

For the subsequent regeneration steps of the germinated embryos until the development of the entire plants, the protocol described by Bertini et al. (2019) was followed, but here other protocols known from the state of art are also applicable since they are even less delicate and critical steps.

The sequence analysis of the DNA extracted from the two plants shows that in both cases the RNP complex was capable of generating the mutation exactly at the target site four nucleotides from the PAM sequence within the gRNA sequence. In the case of RNP1, the mutation involved the insertion of an adenine (A), while in the case of RNP2, the mutation involved the insertion of a thymine (T), causing a frame shift in both cases. SEQ. 3 indicates the starting DNA sequence and SEQ. 4 the sequence with the mutation for the RNP1 case, while SEQ. 5 indicates the starting DNA sequence and SEQ. 6 the sequence with the mutation for the RNP2 case.

Using the protocol according to the invention, in particular the part concerning the transfection of protoplasts and optionally also the part concerning the cultivation of protoplasts modified according to Bertini, the regeneration efficiency of the transfected protoplasts was comparable with that reported for non-transfected protoplasts. From the UV light analysis at the cotyledonary stage, for both transfections (RNP1 and RNP2), one embryo out of about 30 showed fluorescence signal loss which was also maintained at the level of the entire regenerated plant.

The biological material of plant origin used is a table grapevine of the species *Vitis vinifera* subsp. *vinifera* with the name *Sultanina* or *Thomson Seedless* widely present and available on the markets, since it is one of the most important table grapes. Its number in the *Vitis International Variety Catalogue* (VIVC) is **12051**:
https://www.vivc.de/index.php?r=passport%2Fview&id=12051

In the present case, the plants from which the biological material was taken were cultivated in Italy.

The same protoplast transfection and regeneration protocol described for the table variety *Thompson Seedless* is also used for *Chardonnay,* one of the world's most popular wine varieties, number **2455** in the *Vitis International Variety Catalogue.* Also in this case the used plant material was cultivated in Italy. The target gene here is MLO17, a powdery mildew susceptibility gene (Feechan et al., 2008). The timing of the regeneration steps is comparable to that described for *Thompson Seedless* and as disclosed for the method according to the invention.

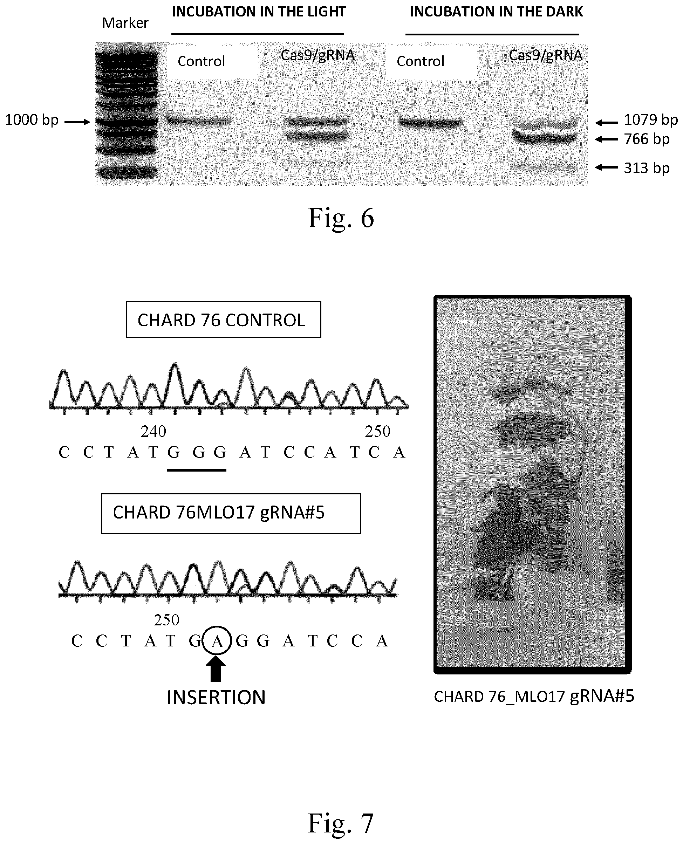

The sequencing of the gene at the gRNA target site (5'-GGAGTTGATGGATCCCAT **AGG-**3' = SEQ. 7; the PAM sequence is highlighted in bold) revealed the insertion of a 3-base-pair adenine, confirming the occurrence of genome editing. Figure 7 shows the adenine insertion in the edited sequence (CCTATGAGGATCCA = SEQ. 9) with respect to the control sequence (CCTATGGGATCCATCA = SEQ. 8) and the Chardonnay plant obtained from the corresponding edited protoplasts.

In order to show whether the dark incubation of the Cas9-gRNA complex before transfection, introduced in the patent application protocol, actually favors the assembly of the complex itself and thus the efficiency of the reaction, an *in-vitro* cut was conducted using the gene sequence encoding the GFP protein and guide RNA 2 as a template.

The assembly of the two components was carried out under light and dark conditions at room temperature for 10 minutes following the protocol described in New England BioLabs inc. (https://international.neb.com/protocols/2014/05/01/in-vitro-digestion-of-dna-with-cas9-nuclease-s-pyogenes-m0386).

After incubation, the Cas9-gRNA complexes were added to the template for 30 minutes at 37°C. Cas9 and gRNA2 were used at a concentration of 30 nM and the template at a concentration of 5 nM.

At the end of the incubation period, the outcome of *in-vitro* cutting was analysed by agarose gel electrophoresis (Figure 6).

The results clearly show that template cutting occurs more efficiently when the complex is assembled in the dark as the uncut templated band (1079 bp) is less intense than when the components are assembled in the light and the bands corresponding to the fragments obtained by cutting (766 bp and 313 bp) are more intense.

For further support, the Image Lab 6.1 software was used, which allowed a quantification of the intensity of the bands after incubation relative to the 1000 bp marker band (Table 1).

**Table 1**

| | Light | Dark |
|---|---|---|
| Template 1079 bp | 22 ng | 16 ng |
| Fragment 766 bp | 21 ng | 28 ng |
| Fragment 313 bp | 5 ng | 10 ng |

During implementation, further embodiment modifications or variants of the method of the invention and the cells or plants which can be obtained therefrom not described herein can be introduced. If such modifications or such variants should fall within the scope of the following claims, they should all be considered protected under the present patent.

### REFERENCES

Bertini, Edoardo; Tornielli, Giovanni Battista; Pezzotti, Mario; Zenoni, Sara; Regeneration of plants from embryogenic callus-derived protoplasts of Garganega and Sangiovese grapevine (Vitis vinifera L.) cultivars, Plant Cell, Tissue and Organ Culture (2019) 138: 239-246 (doi: 10.1007/s 11240-019-01619-1).
Dhekney, Sadanand A.; Li, Zhijian T.; Compton, Michael E.; Gray, Dennis J.; Optimizing Initiation and Maintenance of Vitis Embryogenic Cultures, HortScience, (2009) 44(5): 1400-1406.
Feechan Angela; Jermakow, Angelica M.; Torregrosa, Laurent; Panstruga, Ralph; Dry, Ian B.; Identification of grapevine MLO gene candidates involved in susceptibility to powdery mildew, Functional Plant Biology, (2008) 35: 1255-1266.
Li, Zhijian T.; Dhekney, S. A.; Dutt, M.; Gray, D. J.; An improved protocol for Agrobacterium-mediated transformation of grapevine (Vitis vinifera L.), Plant Cell Tiss Organ Cult, (2008) 93: 311-321 (doi: 10.1007/s11240-008-9378-9).
Malnoy, Mickael; Viola, Roberto; Jung, Min-Hee; Koo, Ok-Jae; Kim, Seokjoong; Kim, Jin-Soo; Velasco, Riccardo; Kanchiswamy, Chidananda Nagamangala; DNA-Free Genetically Edited Grapevine and Apple Protoplast Using CRISPR/Cas9 Ribonucleoproteins, frontiers in Plant Science, (2016) 7:articolo 1904 (doi: 10.3389/fpls.2016.01904).
Osakabe, Yuriko; Liang, Zhenchang; Ren, Chong; Nishitani, Chikako; Osakabe, Keishi; Wada, Masato; Komori, Sadao; Malnoy, Mickael; Velasco, Riccardo; Ploi, Michele; Jung, Min-Hee; Koo, Ok-Jae; Viola, Roberto; Kanchiswamy, Chidananda Nagamangala; CRISPR-Cas9-mediated genome editing in apple and grapevine, nature protocols, (2019) 13: 2844-2863 (doi: 10.1038/s41596-018-0067-9).
Park Sung-Chul; Park, Suhyun; Jeong, Yu Jeong; Lee, Saet Buyl; Pyun, Jang Won; Kim, Soyoung; Kim, Tae Hee; Kim, Suk Weon; Jeong, Jae Cheol; Kim, Cha Young; DNA-free mutagenesis of GIGANTEA in Brassica oleracea var. capitata using CRISPR/Cas9 ribonucleoprotein complexes, Plant Biotechnology Reports, (2019) 13: 483-489.
Reustle, Götz; Harst, Margit; Alleweldt, Gerhardt; Plant regeneration of grapevine (Vitis sp.) protoplasts isolated from embryogenic tissue, Plant Cell Reports (1995) 15:238-241.
Scintilla, Simone; Salvagnin, Umberto; Giacomelli, Lisa; Zeilmaker, Tieme; Malnoy, Mickael A.; van der Voort, Jeroen Rouppe; Moser, Claudio; Regeneration of Plants from DNA-free Edited Grapevine Protoplasts, bioRxiv preprint (2021) (doi: 10.1101/2021.07.16.452503).
Woo, Je Wook; Kim, Jungeun; Kwon, Soon Il; Corvalán, Claudia; Cho, Seung Woo; Kim, Hyeran; Kim, Sang-Gyu; Kim, Sang-Tae; Choe, Sunghwa; Kim, Jin-Soo; DNA-free genome editing in plants with preassembled CRISPR-Cas9 ribonucleoproteins, nature biotechnoilogy, (2015) 33(11): 1162-1164 (doi: 10.1038/nbt3389).
Zhu, Yan-Ming; Hoshino, Yoichiro; Nakano, Masaru; Takahashi, Eikichi; Mii, Masahiro; Highly efficient system of plant regeneration from protoplast of grapevine (Vitis vinifera L.) through somatic embryogenesis by using embryogenic callus culture and activated charcoal, Plant Science 123 (1997) 151-157.

In the sequence listing SEQ. 1 and 2 report target DNAs for GFP guide RNA, while SEQ. 7 reports a target DNA for MLO17 guide RNA.SEQ. 3, 5 and 8 report *Vitis vinifera* DNA and SEQ. 4, 6 and 8 report CRISPR/Cas9 edited *Vitis vinifera* DNA.

## Claims

1. Method for transfection of protoplasts from grapevine plants comprising the following steps:
(I) preparation of the Cas9:gRNA complex with a molar ratio of 1:3 to 3:1, in particular with a weight ratio between 1:1 and 3:1, and incubation of the ribonucleoprotein complex in the dark;
(II) addition of the Cas9:gRNA complex prepared in the previous step to the protoplasts;
(III) addition of a PEG 3000 - PEG 5000, preferably PEG 4000 solution to the mixture obtained in step (II) and incubation, preferably at room temperature, in the dark;
(IV) addition of a salt solution containing MES, in particular a W5 solution, stirring and incubation, preferably at room temperature, and preferably in the dark;
(V) at least one repetition of step (IV), in particular with a doubled volume of saline solution;
(VI) centrifugation of the transfected protoplasts washed in the previous steps and removal of the supernatant;
(VII) proceed with cultivation of the protoplasts without further incubation with incubation periods exceeding 10 min, in particular without any incubation.

2. Method according to claim 1 **characterized in that** the final volume of the mixture of phase (I) is 15-25 µL and/or that the incubation time in phase (I) is 10 min.

3. Method according to claim 1 or 2 **characterized in that** the protoplasts for addition of the Cas9-gRNA complex are resuspended in a concentration of 2 x 10⁵ protoplasts in 200 µL of a MMG solution and that in step (III) 200 - 225 µL, in particular 200 µL of PEG 3000 - PEG 5000 solution, preferably PEG 4000, preferably at 40% by weight by volume, is added.

4. Method according to claim 3, **characterized in that** in step (IV) a volume of saline solution containing MES is added, in particular a W5 solution, which is twice the volume of the MMG solution and that the incubation time is about 8 - 12 min, in particular 10 min.

5. Method according to any one of the preceding claims, **characterized in that** in step (VII) a concentration of 1x10⁵ ppt/mL on *petri* is used.

6. Method according to any one of the preceding claims, **characterized in that** said protoplasts are obtained from the following steps:
(0-1) obtaining embryogenic calluses from apical leaves of plants grown *in vitro*;
(0-2) proliferation of embryogenic calluses and regeneration of somatic embryos;
(0-3) optionally transformation of embryos with *Agrobacterium tumefaciens* in order to introduce a reporter gene, in particular the reporter gene encoding green fluorescent protein (GFP);
(0-4) from the embryos, optionally from the transgenic embryos, induction of the embryogenic callus and isolation of protoplasts.

7. Method according to any one of the preceding claims **characterized in that** for regenerating grapevine plants from the transfected protoplasts the following preparatory steps are added:
(VIII) to the transfected protoplasts is added a culture medium, preferably of the Nitsch type, wherein the liquefied medium is added preferably at a temperature above 35°C and below 48°C;
(IX) the same volume of liquid culture medium, preferably of the Nitsch type, is added to the culture medium after solidification;
(X) incubation of the protoplasts, preferably at 27-28°C, in the dark until regeneration of embryos at the cotyledonary stage;
(XI) germination of the embryos and subsequent development of the seedlings.

8. Method according to claim 7 **characterized in that** in step (VIII) 2 mL of culture medium is added to 800-µL of the protoplast solution and the medium is separated into two halves, after solidification the same amount of culture medium is added as a reserve, and **in that** during step (VIII) and (IX) the culture medium is preferably of the Nitsch type supplemented with 2 mg/L 1-naphthaleneacetic acid (NAA), 0.5 mg/L 6-benzylaminopurine (6-BAP), 0.3 M glucose, 0.09 M sucrose and 2 g/L gellan gum (pH 5.7) wherein in step (IX) the medium was supplemented with 0.3% activated charcoal or similar, e.g. glutathione; that during step (X) the liquid medium was replaced every 10-20 days, preferably every 12-18 days with fresh medium as defined above but without glucose, that after 3-4 months of culture, the somatic embryos at the cotyledonary stage derived from protoplasts, were transferred to a solid preferably of the Nitsch type supplemented with 30 g/L sucrose and 2 g/L gellan gum (pH 5.7) and kept in the dark for 3-5 weeks, preferably 4-5 weeks to allow complete germination.

## Patentansprüche

1. Methode zur Transfektion von Protoplasten aus Weinrebenpflanzen, umfassend die folgenden Schritte:
(I) Herstellung des Cas9:gRNA-Komplexes in einem molaren Verhältnis von 1:3 bis 3:1, insbesondere in einem Gewichtsverhältnis von 1:1 bis 3:1, und Inkubation des Ribonukleoprotein-Komplexes im Dunkeln;
(II) Zugabe des im vorhergehenden Schritt (I) hergestellten Cas9:gRNA-Komplexes zu den Protoplasten;
(III) Zugabe einer Lösung aus PEG 3000 - PEG 5000, vorzugsweise PEG 4000, zu dem in Schritt (II) erhaltenen Gemisch und Inkubation, vorzugsweise bei Raumtemperatur, im Dunkeln;
(IV) Zugabe einer Salzlösung, die MES enthält, insbesondere einer W5-Lösung, sowie Rühren und Inkubation, vorzugsweise bei Raumtemperatur, und vorzugsweise im Dunkeln;
(V) mindestens eine Wiederholung von Schritt (IV), insbesondere mit einem verdoppelten Volumen der Salzlösung;
(VI) Zentrifugation der in den vorherigen Schritten gewaschenen transfizierten Protoplasten und Entfernung des Überstands;
(VII) Weiterkultivierung der Protoplasten, ohne eine weitere Inkubation mit Inkubationszeiten von mehr als 10 Minuten, insbesondere ohne jegliche Inkubation.

2. Methode gemäß Anspruch 1, **dadurch gekennzeichnet, dass** das Endvolumen der Mischung der Phase (I) 15-25 µl beträgt und/oder dass die Inkubationszeit in Phase (I) 10 Minuten beträgt.

3. Verfahren nach Anspruch 1 oder 2, **dadurch gekennzeichnet, dass** die Protoplasten für die Zugabe des Cas9-gRNA-Komplexes in einer Konzentration von 2 × 10⁵ Protoplasten in 200 µL einer MMG-Lösung resuspendiert werden und dass in Schritt (III) 200-225 µL, insbesondere 200 µL PEG 3000 - PEG 5000-Lösung, vorzugsweise PEG 4000, vorzugsweise in einer Konzentration von 40 Gew.-%, zugegeben werden.

4. Methode gemäß Anspruch 3, **dadurch gekennzeichnet, dass** in Schritt (IV) ein Volumen einer MES-haltigen Salzlösung, insbesondere einer W5-Lösung, zugegeben wird, das doppelt so groß ist wie das Volumen der MMG-Lösung, und dass die Inkubationszeit etwa 8 bis 12 Minuten, insbesondere 10 Minuten, beträgt.

5. Methode gemäß einem beliebigen der vorstehenden Ansprüche, **dadurch gekennzeichnet, dass** in Schritt (VII) eine Konzentration von 1x10⁵ ppt/mL auf Petrischalen verwendet wird.

6. Methode gemäß einem beliebigen der vorstehenden Ansprüche, **dadurch gekennzeichnet, dass** besagte Protoplasten aus den folgenden Schritten gewonnen werden:
(0-1) Gewinnung embryogener Kallusgewebe aus apikalen Blättern von *in vitro* gezüchteten Pflanzen;
(0-2) Vermehrung der embryogenen Kallusgewebe und Regeneration somatischer Embryonen;
(0-3) wahlweise Transformation der Embryonen mit *Agrobacterium tumefaciens*, um ein Reportergen, insbesondere das für grün fluoreszierendes Protein (GFP) kodierende Reportergen, einzuführen;
(0-4) aus den Embryonen, wahlweise aus den transgenen Embryonen, Induktion des embryogenen Kallusgewebes und Isolierung der Protoplasten.

7. Methode gemäß einem beliebigen der vorstehenden Ansprüche, **dadurch gekennzeichnet, dass** zur Regenerierung von Weinrebenpflanzen aus den transfizierten Protoplasten die folgenden vorbereitenden Schritte hinzugefügt werden:
(VIII) zu den transfizierten Protoplasten wird ein Kulturmedium, vorzugsweise vom Typ Nitsch, hinzugefügt, wobei das verflüssigte Medium vorzugsweise bei einer Temperatur über 35 °C und unter 48 °C hinzugefügt wird;
(IX) dem Kulturmedium wird nach der Verfestigung das gleiche Volumen an flüssigem Kulturmedium, vorzugsweise vom Typ Nitsch, hinzugefügt;
(X) Inkubation der Protoplasten, vorzugsweise bei 27-28 °C, im Dunkeln bis zur Regeneration von Embryonen im Keimblattstadium;
(XI) Keimung der Embryonen und anschließende Entwicklung der Sämlinge.

8. Methode gemäß Anspruch 7, **dadurch gekennzeichnet, dass** in Schritt (VIII) 2 mL Kulturmedium zu 800 µL der Protoplastenlösung gegeben werden und das Medium in zwei Hälften geteilt wird, nach der Verfestigung die gleiche Menge Kulturmedium als Reserve hinzugefügt wird und dass während der Schritte (VIII) und (IX) das Kulturmedium vorzugsweise vom Nitsch-Typ ist, ergänzt mit 2 mg/L 1-Naphthalinessigsäure (NAA), 0,5 mg/L 6-Benzylaminopurin (6-BAP), 0,3 M Glucose, 0,09 M Saccharose und 2 g/L Gellangummi (pH 5,7), wobei in Schritt (IX) das Medium mit 0,3 % Aktivkohle oder ähnlichem, z. B. Glutathion, ergänzt wurde; dass in Schritt (X) das flüssige Medium alle 10-20 Tage, vorzugsweise alle 12-18 Tage, durch frisches Medium, wie oben definiert, jedoch ohne Glucose, ersetzt wurde, dass nach 3-4 Monaten Kultur die aus Protoplasten gewonnenen somatischen Embryonen im Keimblattstadium auf ein festes Medium, vorzugsweise vom Typ Nitsch, übertragen wurden, das mit 30 g/L Saccharose und 2 g/L Gellangummi (pH 5,7) ergänzt war, und 3-5 Wochen, vorzugsweise 4-5 Wochen, im Dunkeln belassen wurden, um eine vollständige Keimung zu ermöglichen.

## Revendications

1. Méthode de transfection de protoplastes de plants de vigne comprenant les étapes suivantes:
(I) préparation du complexe Cas9:gRNA avec un rapport molaire de 1:3 à 3:1, en particulier avec un rapport pondéral compris entre 1:1 et 3:1, et incubation du complexe ribonucléoprotéique dans l'obscurité;
(II) ajout du complexe Cas9:gRNA préparé à l'étape précédente aux protoplastes;
(III) ajout d'une solution de PEG 3000 - PEG 5000, de préférence PEG 4000, au mélange obtenu à l'étape (II) et incubation, de préférence à température ambiante, dans l'obscurité;
(IV) ajout d'une solution saline contenant du MES, en particulier une solution W5, agitation et incubation, de préférence à température ambiante, et de préférence dans l'obscurité;
(V) au moins une répétition de l'étape (IV), en particulier avec un volume doublé de solution saline;
(VI) centrifugation des protoplastes transfectés lavés lors des étapes précédentes et élimination du surnageant;
(VII) poursuite de la culture des protoplastes sans incubation supplémentaire avec des périodes d'incubation supérieures à 10 minutes, en particulier sans aucune incubation.

2. Méthode selon la revendication 1, **caractérisée en ce que** le volume final du mélange de la phase (I) est de 15 à 25 µL et/ou **en ce que** le temps d'incubation dans la phase (I) est de 10 minutes.

3. Méthode selon la revendication 1 ou 2, **caractérisée en ce que** les protoplastes destinés à l'ajout du complexe Cas9:gRNA sont remis en suspension à une concentration de 2 x 10⁵ protoplastes dans 200 µL d'une solution MMG et **en ce que**, à l'étape (III), 200 à 225 µL, en particulier 200 µL de PEG 3000 - PEG 5000, de préférence du PEG 4000, de préférence à 40 % en poids par volume, sont ajoutés.

4. Méthode selon la revendication 3, **caractérisée en ce que**, à l'étape (IV), on ajoute un volume de solution saline contenant du MES, en particulier une solution W5, qui est deux fois plus grande que le volume de la solution MMG, et **en ce que** le temps d'incubation est d'environ 8 à 12 minutes, en particulier 10 minutes.

5. Méthode selon l'une quelconque des revendications précédentes, **caractérisée en ce q**ue, à l'étape (VII), on utilise une concentration de 1x10⁵ ppt/mL sur une boîte de Pétri.

6. Méthode selon l'une quelconque des revendications précédentes, **caractérisée en ce que** lesdits protoplastes sont obtenus à partir des étapes suivantes:
(0-1) obtention de callosités embryogènes à partir des feuilles apicales de plantes cultivées *in vitro*;
(0-2) prolifération des callosités embryogènes et régénération d'embryons somatiques;
(0-3) transformation optionnelle des embryons avec *Agrobacterium tumefaciens* afin d'introduire un gène rapporteur, en particulier le gène rapporteur codant pour la protéine fluorescente verte (GFP);
(0-4) à partir des embryons, optionnellement à partir des embryons transgéniques, induction de la callosité embryogène et isolement des protoplastes.

7. Méthode selon l'une quelconque des revendications précédentes, **caractérisée en ce que**, pour régénérer des plants de vigne à partir des protoplastes transfectés, les étapes préparatoires suivantes sont ajoutées:
(VIII) on ajoute aux protoplastes transfectés un milieu de culture, de préférence de type Nitsch, dans lequel le milieu liquéfié est ajouté de préférence à une température supérieure à 35 °C et inférieure à 48 °C;
(IX) le même volume de milieu de culture liquide, de préférence de type Nitsch, est ajouté au milieu de culture après solidification;
(X) incubation des protoplastes, de préférence à 27-28 °C, dans l'obscurité jusqu'à la régénération des embryons au stade cotylédon;
(XI) germination des embryons et développement ultérieur des plantules.

8. Méthode selon la revendication 7, **caractérisée en ce que,** à l'étape (VIII), 2 mL de milieu de culture sont ajoutés à 800 µL de la solution de protoplastes et le milieu est séparé en deux moitiés, après solidification, la même quantité de milieu de culture est ajoutée en réserve, et **en ce que** pendant les étapes (VIII) et (IX), le milieu de culture est de préférence du type Nitsch complété par 2 mg/L d'acide 1-naphtalèneacétique (NAA), 0,5 mg/L de 6-benzylaminopurine (6-BAP), 0,3 M de glucose, 0,09 M de saccharose et 2 g/L de gomme gellane (pH 5,7), où, à l'étape (IX), le milieu a été additionné de 0,3 % de charbon actif ou similaire, par exemple du glutathion; au cours de l'étape (X), le milieu liquide a été remplacé tous les 10 à 20 jours, de préférence tous les 12 à 18 jours, par un milieu frais tel que défini ci-dessus, mais sans glucose, après 3 à 4 mois de culture, les embryons somatiques au stade cotylédonaire dérivés de protoplastes ont été transférés sur un milieu solide, de préférence de type Nitsch, additionné de 30 g/L de saccharose et de 2 g/L de gomme gellane (pH 5,7), et conservés dans l'obscurité pendant 3 à 5 semaines, de préférence 4 à 5 semaines, pour permettre une germination complète.
